# EUROPÄISCHE PATENTSCHRIFT

(11) **EP 3 071 251 B1**
(45) Veröffentlichungstag und Bekanntmachung des Hinweises auf die Patenterteilung: **13.03.2019**
(21) Anmeldenummer: 14799153.3
(22) Anmeldetag: 14.11.2014
(51) Int. Cl.: A61M 1/16

(54) **VORRICHTUNG ZUM BILANZIEREN VON FLÜSSIGKEITEN FÜR EINE EXTRAKORPORALE BLUTBEHANDLUNGSVORRICHTUNG**
DEVICE FOR BALANCING LIQUIDS FOR AN EXTRACORPOREAL BLOOD TREATMENT DEVICE
DISPOSITIF D'ÉQUILIBRAGE DE FLUIDES POUR UN DISPOSITIF DE TRAITEMENT EXTRACORPOREL DU SANG

(30) Priorität: 19.11.2013 DE 102013019356
(43) Veröffentlichungstag der Anmeldung: 28.09.2016
(73) Patentinhaber: Fresenius Medical Care Deutschland GmbH, 61352 Bad Homburg (DE)
(72) Erfinder: PETERS, Arne, 61352 Bad Homburg (DE); WIKTOR, Christoph, 63571 Gelnhausen (DE)
(74) Vertreter: Oppermann, Frank
(86) Internationale Anmeldenummer: PCT/EP2014/074684
(87) Internationale Veröffentlichungsnummer: WO 2015/074973

(56) Entgegenhaltungen:
- EP-A1- 0 516 152
- EP-A1- 2 447 684
- US-A- 4 267 040
- US-A1- 2007 038 191

## Beschreibung

Die Erfindung betrifft eine Vorrichtung zum Bilanzieren von Flüssigkeiten für eine extrakorporale Blutbehandlungsvorrichtung mit einer Blutbehandlungseinheit, insbesondere eine Dialysevorrichtung mit einem Dialysator.
Die bekannten Dialysevorrichtungen verfügen über einen Dialysator oder Filter, der durch eine semipermeable Membran in eine Blutkammer und eine Dialysierflüssigkeitskammer getrennt ist. Während der Dialysebehandlung strömt das Blut des Patienten in einem extrakorporalen Blutkreislauf durch die Blutkammer des Dialysators, während die Dialysierflüssigkeit in einem Dialysierflüssigkeitskreislauf durch die Dialysierflüssigkeitskammer des Dialysators strömt.
Wegen der großen Austauschmengen ist eine exakte Bilanzierung der dem Patienten entzogenen Flüssigkeit und der dem Patienten zugeführten Flüssigkeit über die gesamte Behandlungszeit erforderlich. Zum Stand der Technik gehören gravimetrische und volumetrische Bilanziervorrichtungen.
Die volumetrischen Bilanziervorrichtungen verfügen über eine Bilanziereinheit, die eine durch eine bewegliche Trennwand in zwei Kompartimente unterteilte Bilanzkammer aufweist. Die beiden Kompartiments werden über Zuführ- bzw. Abführleitungen wechselweise mit frischer bzw. verbrauchter Dialysierflüssigkeit befüllt, so dass frische und verbrauchte Dialysierflüssigkeit bilanziert werden. Zur Herstellung eines kontinuierlichen Flusses von Dialysierflüssigkeit durch die Dialysierflüssigkeitskammer des Dialysators werden in der Praxis zwei Bilanzkammern parallel geschaltet. Eine Bilanziervorrichtung mit einer Bilanzkammer ist beispielsweise aus der DE 26 34 238 A1 und mit zwei Bilanzkammern aus der DE 28 38 414 A1 bekannt.

Die WO 2011/157396 A1 beschreibt eine Dialysevorrichtung, die über einen inneren Dialysierflüssigkeitskreislauf, der den Dialysator einschließt, und einen äußeren Dialysierflüssigkeitskreislauf verfügt, dem frische Dialysierflüssigkeit zugeführt bzw. aus dem verbrauchte Dialysierflüssigkeit abgeführt wird. Die Zu- und Abfuhr von frischer bzw. verbrauchter Dialysierflüssigkeit in den bzw. aus dem äußeren Dialysierflüssigkeitskreislauf kann mit einer anderen Flussrate erfolgen, als die Flussrate, mit der die Dialysierflüssigkeit in dem inneren Dialysierflüssigkeitskreislauf über den Dialysator zirkuliert.

Aus der US 2007/0038191 A1 ist eine Dialysevorrichtung bekannt, die über eine Blutbehandlungseinheit und eine Bilanziereinheit verfügt. Die Dialysevorrichtung weist eine zu der Bilanziereinheit führende erste Zuflussleitung zum Zuführen von Flüssigkeit in die Bilanziereinheit und eine von der Bilanziereinheit abgehende erste Abflussleitung zum Abführen von Flüssigkeit aus der Bilanziereinheit auf. Von der Bilanziereinheit geht eine zweite Abflussleitung zum Fördern von Flüssigkeit aus der Bilanziereinheit in die Blutbehandlungseinheit ab und zu der Bilanziereinheit führt eine zweite Zuflussleitung zum Fördern von Flüssigkeit aus der Blutbehandlungseinheit in die Bilanziereinheit.

Die Bilanziereinheit weist zwei identische Bilanzkammern auf, die von einer Membran jeweils in zwei Bilanzkammerhälften unterteilt sind. Die volumetrische Bilanzierung erfolgt dadurch, dass die identischen Bilanzkammerhälften unter Verwerfung von frischer bzw. gebrauchter Dialysierflüssigkeit wechselweise befüllt bzw. entleert werden. Die Dialysevorrichtung sieht für die volumetrische Bilanzierung eine Überwachungseinrichtung vor, die eine Waage aufweist, die das Gesamtgewicht der beiden Bilanzkammerhälften einer Bilanzkammer misst. Wenn das Gesamtgewicht oberhalb oder unterhalb eines Grenzwertes liegt, kann ein Alarm gegeben werden.

Die EP 0 516 152 A1 beschreibt eine Blutbehandlungsvorrichtung mit einer Bilanziereinheit, die auf der Überwachung der Differenz des Volumenstroms der in den Dialysator fließenden und aus dem Dialysator fließenden Dialysierflüssigkeit beruht. Die Bilanziereinheit weist eine Steuereinheit zum Einstellen der Förderarten der Pumpen in der Zufluss- bzw. Abflussleitung auf.

Aus der US 4 267 040 A ist eine Blutbehandlungsvorrichtung mit einer Bilanziereinheit bekannt, die zwei Bilanzkammern aufweist, die jeweils in zwei Bilanzkammerhälften unterteilt sind. Die Blutbehandlungsvorrichtung sieht eine Dosierung von Konzentrat zur Herstellung der Dialysierflüssigkeit vor. Das Konzentrat wird dem Wasser mittels einer Dosiereinrichtung in der Zuflussleitung zugeführt, wobei die Zusammensetzung der Dialysierflüssigkeit in der Zuflussleitung mittels einer Analyseeinrichtung überwacht wird. Dabei soll die Bilanziereinheit sicherstellen, dass bei der Bilanzierung die Zufuhr von Konzentrat Berücksichtigung findet.

Die EP 2 447 684 A1 beschreibt eine Einrichtung zur Überwachung des Gewichts von Behältern zur Aufnahme von Filtrat und Substituat für eine Blutbehandlungsvorrichtung. Die Gewichtsüberwachungseinrichtung misst das Gesamtgewicht des Filtrat- und Substituatsbehälters mit zwei unabhängigen Messeinrichtungen, die unabhängig voneinander arbeiten. Das mit den beiden Waagen gemessene Gewicht wird miteinander verglichen. Wenn die gemessenen Gewichte gleich sind, wird auf eine ordnungsgemäße Funktion geschlossen. Ansonsten wird darauf geschlossen, dass die Gewichtsmessung fehlerhaft ist.

Der Erfindung liegt die Aufgabe zu Grunde, die Gefahr einer fehlerhaften Bilanzierung während einer extrakorporalen Blutbehandlung zu verringern und somit die Sicherheit der extrakorporalen Blutbehandlung zu erhöhen.

Die Lösung dieser Aufgabe erfolgt erfindungsgemäß mit den Merkmalen der unabhängigen Patentansprüche. Die Gegenstände der abhängigen Ansprüche betreffen vorteilhafte Ausführungsformen der Erfindung.

Die erfindungsgemäße Vorrichtung beruht auf der Überwachung von einem inneren Flüssigkeitssystem, das die Blutbehandlungseinheit einschließt, und einem äußeren Flüssigkeitssystem, dem frische Flüssigkeit zugeführt bzw. aus dem verbrauchte Flüssigkeit abgeführt wird. Zur Überwachung der Bilanzierung von frischer und verbrauchter Flüssigkeit in dem inneren Flüssigkeitssystem wird die Zu- und Abfuhr von frischer bzw. verbrauchter Flüssigkeit in das bzw. aus dem äußeren Flüssigkeitssystem überwacht.
Die erfindungsgemäße Vorrichtung zum Bilanzieren von Flüssigkeiten verfügt über eine Bilanziereinheit, die eine oder zwei Bilanzkammern aufweisen kann. Dabei ist unerheblich, wie die Bilanzkammern ausgebildet sind. Die Bilanzkammern können ein Kompartiment, das frische und verbrauchte Flüssigkeit aufnimmt oder zwei Kompartimente umfassen, die durch eine Trennwand getrennt sind.

Die Bilanziereinheit stellt sicher, dass der Blutbehandlungseinheit ein vorgegebenes Volumen an Flüssigkeit zugeführt wird bzw. aus der Blutbehandlungseinheit ein vorgegebenes Volumen an Flüssigkeit abgeführt wird.
Zur Versorgung der Bilanziereinheit mit frischer bzw. verbrauchter Dialysierflüssigkeit ist eine zu der Bilanziereinheit führende erste Zuflussleitung zum Zuführen von frischer Flüssigkeit und eine von der Bilanziereinheit abgehende erste Abflussleitung zum Abführen von Flüssigkeit vorgesehen. Von der Bilanziereinheit geht eine zweite Abflussleitung zum Fördern von frischer Flüssigkeit aus der Bilanziereinheit in die Blutbehandlungseinheit ab und von der Blutbehandlungseinheit führt eine zweite Zuflussleitung zum Fördern von Flüssigkeit aus der Blutbehandlungseinheit in die Bilanziereinheit.
Die erfindungsgemäße Bilanziervorrichtung verfügt über eine Überwachungseinrichtung zur Erkennung einer fehlerhaften Bilanzierung von der Flüssigkeit, die der Blutbehandlungseinheit zugeführt wird, und der Flüssigkeit, die aus der Blutbehandlungseinheit abgeführt wird. Die Überwachungseinrichtung weist eine Recheneinheit auf, die derart konfiguriert ist, dass das Volumen oder eine mit dem Volumen korrelierende Größe der Flüssigkeit, die der Bilanziereinheit zugeführt wird, mit dem Volumen oder einer mit dem Volumen korrelierenden Größe der Flüssigkeit, die aus der Bilanziereinheit abgeführt wird, ins Verhältnis gesetzt wird. Auf der Grundlage des Verhältnisses des Volumens oder der mit dem Volumen korrelierenden Größe von der Bilanziereinheit zugeführter und abgeführter Flüssigkeit wird auf eine fehlerhafte Bilanzierung geschlossen.

Die erfindungsgemäße Vorrichtung beruht darauf, dass eine fehlerhafte Bilanzierung dann vorliegt, wenn das Verhältnis des Volumens oder der mit dem Volumen korrelierenden Größe von der Bilanziereinheit zugeführter und aus der Bilanziereinheit abgeführter Flüssigkeit nicht einem vorgegebenen Wert entspricht. Von einer fehlerfreien Bilanzierung wird grundsätzlich dann ausgegangen, wenn das Volumen oder die mit dem Volumen korrelierende Größe der zugeführten Flüssigkeit dem Volumen oder der mit dem Volumen korrelierenden Größe der abgeführten Flüssigkeit entspricht. Wenn sich die Volumina nicht einander entsprechen, wird davon ausgegangen, dass eine Störung vorliegt. Wenn beispielsweise in dem Flüssigkeitssystem eine Leckage auftritt, können die Volumina von zugeführter und abgeführter Flüssigkeit nicht einander entsprechen. Die von der Bilanzierung mit der Bilanziereinheit unabhängige Überwachung der Flüssigkeitszufuhr bzw. -abfuhr stellt eine Plausibilitätsprüfung dar, mit der die Gefahr von Fehlbilanzierungen weiter verringert und somit die Sicherheit der Blutbehandlung weiter erhöht wird.
Zur Bestimmung des Volumens von zu- bzw. abgeführter Flüssigkeit kann der Durchfluss der Flüssigkeit in den Leitungen gemessen werden. Anstelle der Messung der Flussraten kann auch das Gewicht der Flüssigkeiten als eine mit dem Volumen korrelierende Größe bestimmt werden. Zur Bestimmung des Gewichts der Flüssigkeiten weist die Vorrichtung zum Bilanzieren eine Wiegeeinrichtung auf.

Eine beispielhafte, nicht erfindungsgemäße Ausführungsform sieht ein einziges Behältnis vor, in dem die frische Flüssigkeit bereitgestellt wird und die verbrauchte Flüssigkeit aufgenommen wird. Bei dieser Ausführungsform weist die Wiegeeinrichtung eine Waage und die Überwachungseinrichtung eine Recheneinheit auf, die derart konfiguriert ist, dass auf eine fehlerhafte Bilanzierung geschlossen wird, wenn in einem vorgegebenen Zeitintervall sich das Gewicht des Behältnisses um einen Betrag ändert, der größer als ein vorgegebener Grenzwert ist. Der vorgegebene Grenzwert ist vorzugsweise 0. Folglich wird auf eine fehlerhafte Bilanzierung dann geschlossen, wenn das Gewicht des einzigen Behältnisses zu- bzw. abnimmt. Durch die Vorgabe eines Grenzwertes, der >0 ist, beispielsweise 100g pro Stunde Behandlungszeit oder 500g pro Gesamtbehandlung, kann für die Bilanzierung ein bestimmter Toleranzbereich zugelassen werden.
Eine alternative Ausführungsform sieht vor, dass frische Flüssigkeit in einem ersten Behältnis bereitgestellt wird, während verbrauchte Flüssigkeit in einem zweiten Behältnis gesammelt wird. Bei dieser Ausführungsform kann die Wiegeeinrichtung eine Waage oder zwei Waagen aufweisen. Wenn die nicht erfindungsgemäße Wiegeeinrichtung nur eine Waage aufweist, werden beide die Behältnisse gewogen. Bei der erfindungsgemäßen Wiegeeinrichtung mit zwei Waagen, werden das eine Behältnis mit der ersten und das andere Behältnis mit der zweiten Waage gewogen.

Bei der Ausführungsform mit den beiden Waagen ist die Recheneinheit der Überwachungseinrichtung derart konfiguriert, dass auf eine fehlerhafte Bilanzierung geschlossen wird, wenn in einem vorgegebenen Zeitintervall das Gewicht des ersten Behältnisses um einen Betrag von dem Gewicht des zweiten Behältnisses abweicht, der größer als ein vorgegebener Grenzwert ist.

Die Messung des Gewichts der Flüssigkeiten setzt voraus, dass die Flüssigkeiten in Behältnissen bereitgestellt bzw. gesammelt werden. Die Behältnisse können starre Behältnisse oder flexible Behältnisse, beispielsweise Folienbeutel sein. Damit ist aber die Menge der Flüssigkeiten begrenzt.

Eine kontinuierliche Zu- und Abfuhr von Flüssigkeiten aus einer zentralen Versorgungseinheit setzt die Überwachung des Durchflusses der Flüssigkeiten voraus. Bei einer weiteren bevorzugten Ausführungsform verfügt die Überwachungseinrichtung über eine Durchfluss-Messeinrichtung, die einen ersten Durchfluss-Sensor in der ersten Zuflussleitung und einen zweiten Durchfluss-Sensor in der ersten Abflussleitung aufweist, über die frische und verbrauchte Flüssigkeit dem äußeren Flüssigkeitssystem zu- bzw. aus dem äußeren Flüssigkeitssystem abgeführt werden. Die Recheneinheit der Überwachungseinrichtung ist bei dieser Ausführungsform derart konfiguriert, dass auf eine fehlerhafte Bilanzierung geschlossen wird, wenn der Durchfluss in der ersten Zuflussleitung um einen vorgegebenen Betrag von dem Durchfluss in der ersten Abflussleitung abweicht. Der vorgegebene Betrag ist vorzugsweise 0. Der Betrag kann aber auch ein Wert >0 sein, beispielsweise 100g pro Stunde Behandlungszeit oder 500g pro Gesamtbehandlung, wenn ein bestimmter Toleranzbereich für die Bilanzierung zugelassen wird.

Wenn auf eine fehlerhafte Bilanzierung geschlossen wird, kann ein Steuersignal erzeugt werden, das einen Alarm auslöst oder die Vornahme eines Eingriffs in die Maschinensteuerung der Blutbehandlungsvorrichtung auslöst.

Eine besonders bevorzugte Ausführungsform sieht eine Ultrafiltrationseinrichtung vor, um dem Patienten mit einer vorgegebenen Ultrafiltrationsrate Flüssigkeit (Ultrafiltrat) entziehen zu können. Die Ultrafiltrationseinrichtung weist eine Ultrafiltrations-Pumpe auf, mit der dem inneren Flüssigkeitssystem ein vorgegebenes Volumen an Ultrafiltrat in einem vorgegebenen Zeitintervall entzogen werden kann. Die Ultrafiltrations-Leitung ist vorzugsweise an die zweite Abflussleitung angeschlossen, über die Flüssigkeit von der Blutbehandlungseinheit zu der Bilanziereinheit gefördert wird. Die Ultrafiltrations-Leitung ist mit dem Behältnis für verbrauchte Flüssigkeit verbunden. Dadurch ist eine Überprüfung des dem Patienten entzogenen Volumens an Flüssigkeit mit der Überwachung des Gewichts desjenigen Behältnisses, dem das Ultrafiltrat zugeführt wird, möglich. Bei einer nicht erfindungsgemäßen Ausführungsform mit einem einzigen Behältnis wird auf eine fehlerhafte Bilanzierung geschlossen, wenn sich das Gewicht des Behältnisses, dem das Ultrafiltrat zugeführt wird, nicht um einen Betrag ändert, der dem Gewicht des Volumens des in dem vorgegebenen Zeitintervall entzogenen Ultrafiltrats entspricht. Bei einer Ausführungsform mit zwei Behältnissen wird auf eine fehlerhafte Bilanzierung geschlossen, wenn das Gewicht des einen Behältnisses, dem Ultrafiltrat zugeführt wird, nicht um einen Betrag von dem Gewicht des anderen Behältnisses abweicht, der dem Gewicht des Volumens des in dem vorgegebenen Zeitintervall entzogenen Ultrafiltrats entspricht.
Die Ultrafiltrations-Pumpe ist vorzugsweise eine Pumpe, die Ultrafiltrat mit einer sehr exakt einzustellenden Förderrate fördert, so dass das Volumen des in dem vorgegebenen Zeitintervall entzogenen Ultrafiltrats exakt bestimmt werden kann. Es ist aber auch möglich, Ultrafiltrat in einem Behältnis zu sammeln und das Gewicht des Ultrafiltrats zu bestimmen.
Im Folgenden werden verschiedene Ausführungsbeispiele der Erfindung unter Bezugnahme auf die Figuren im Einzelnen erläutert.
Es zeigen:
- Fig. 1: eine nicht erfindungsgemäße Dialysevorrichtung mit einer Vorrichtung zum Bilanzieren von frischer und verbrauchter Dialysierflüssigkeit in vereinfachter schematischer Darstellung,
- Fig. 2: ein zweites, nicht erfindungsgemäßes Ausführungsbeispiel der Dialysevorrichtung mit der Bilanziervorrichtung,
- Fig. 3: ein drittes, nicht erfindungsgemäßes Ausführungsbeispiel der Dialysevorrichtung,
- Fig. 4: ein viertes, erfindungsgemäßes Ausführungsbeispiel der Dialysevorrichtung und
- Fig. 5: ein fünftes, erfindungsgemäßes Ausführungsbeispiel der Dialysevorrichtung.

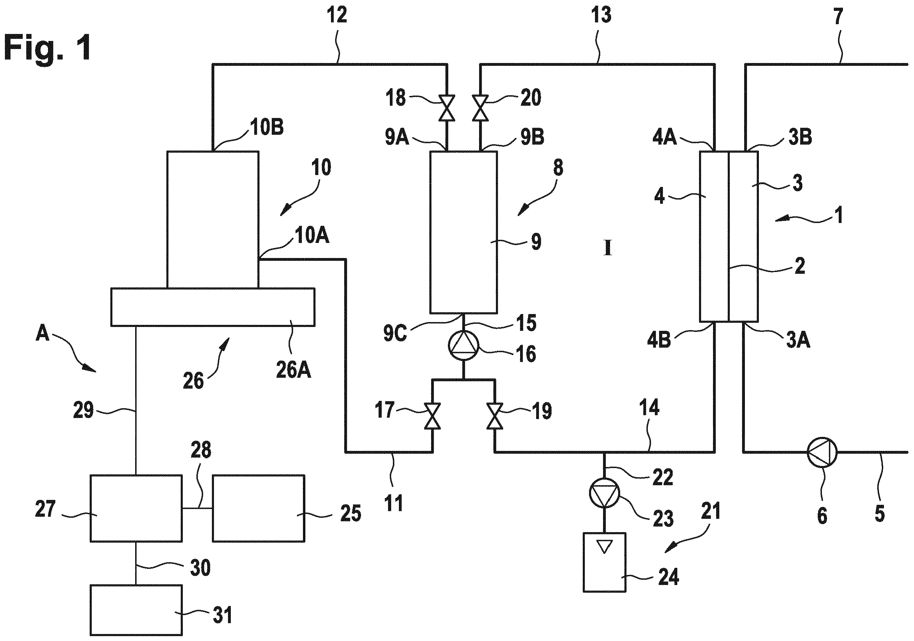

Die Erfindung wird nachfolgend am Beispiel einer Blutbehandlungsvorrichtung beschrieben, die über einen Dialysator als Blutbehandlungseinheit verfügt. Fig. 1 zeigt in stark vereinfachter schematischer Darstellung die wesentlichen Komponenten einer nicht erfindungsgemäßen Dialysevorrichtung. Die Vorrichtung zur Bilanzierung von frischer und verbrauchter Dialysierflüssigkeit ist Bestandteil der Dialysevorrichtung.
Die Dialysevorrichtung weist einen Dialysator 1 auf, der durch eine semipermeable Membran 2 in eine Blutkammer 3 und eine Dialysierflüssigkeitskammer 4 getrennt ist. Von dem Patienten führt eine Blutzuführleitung 5, in die eine Blutpumpe 6 geschaltet ist, zu einem Einlass 3A der Blutkammer 3, während von einem Auslass 3B der Blutkammer 3 eine Blutabführleitung 7 abgeht, die zu dem Patienten führt. Während der Blutbehandlung strömt das Blut des Patienten in dem extrakorporalen Blutkreislauf I durch die Blutkammer 3 des Dialysators 1.

Der Dialysierflüssigkeitskammer 4 des Dialysators 1 wird frische Dialysierflüssigkeit zugeführt und verbrauchte Dialysierflüssigkeit wird aus der Dialysierflüssigkeitskammer 4 abgeführt. Die Bilanzierung von frischer und verbrauchter Dialysierflüssigkeit erfolgt mit der erfindungsgemäßen Bilanziervorrichtung, die Bestandteil der Dialysevorrichtung ist.

Die Bilanziervorrichtung verfügt über eine Bilanziereinheit 8, die bei dem vorliegenden Ausführungsbeispiel eine Bilanzkammer 9 aufweist. Bei dem vorliegenden Ausführungsbeispiel ist die Bilanzkammer 9 ein volumenstarres Behältnis mit einem ersten Auslass 9A und einem zweiten Auslass 9B an der Oberseite und einem gemeinsamen Einlass 9C an der Unterseite.

Frische und gebrauchte Dialysierflüssigkeit werden in einem einzigen Behältnis 10, das ein starres Behältnis aber auch ein flexibler Beutel sein kann, bereitgestellt bzw. gesammelt. Das einzige Behältnis 10 kann durch eine flexible Trennwand in zwei Kompartiments unterteilt sein, die frische von gebrauchter Dialysierflüssigkeit trennt.

Der Auslass 10A des Behältnisses 10 ist über eine erste Zuflussleitung 11 mit dem Einlass 9C an der Unterseite der Bilanzkammer 9 verbunden, während der erste Auslass 9A an der Oberseite der Bilanzkammer 9 über eine erste Abflussleitung 12 mit dem Einlass 10B des Behältnisses 10 verbunden ist. Der zweite Auslass 9B an der Oberseite der Bilanzkammer 9 ist über eine zweite Abflussleitung 13 mit dem Einlass 4A der Blutbehandlungseinheit 4 verbunden, während der Auslass 4B der Blutbehandlungseinheit 4 über eine zweite Zuflussleitung 14 mit dem gemeinsamen Einlass 9C der Bilanzkammer 9 verbunden ist.

Die erste Zuflussleitung 11 und die zweite Zuflussleitung 14 weisen einen gemeinsamen Leitungsabschnitt 15 auf, in den eine Dialysierflüssigkeitspumpe 16 geschaltet ist.

Zum Unterbrechen des Zuflusses von Flüssigkeit in die Bilanziereinheit 8 und/oder Abflusses von Flüssigkeit aus der Bilanziereinheit sind in der ersten Zufluss- und Abflussleitung 11, 12 ein erstes bzw. zweites Absperrorgan 17, 18 und in der zweiten Zufluss- und Abflussleitung 14, 13 ein drittes bzw. viertes Absperrorgan 19, 20 angeordnet.

Die Dialysevorrichtung verfügt weiterhin über eine Ultrafiltrationseinrichtung 21, die eine von der zweiten Zuflussleitung 14 abzweigende Ultrafiltrationsleitung 22 aufweist, in die eine Ultrafiltrationspumpe 23 geschaltet ist. Die Ultrafiltrations-Pumpe 23 fördert mit hoher Genauigkeit mit einer vorgegebenen Ultrafiltrationsrate ein vorgegebenes Volumen an Ultrafiltrat in einem vorgegebenen Zeitintervall in einen Ablauf 24.

Die Dialysevorrichtung weist eine zentrale Steuereinheit 25 auf, die über nicht dargestellte Steuerleitungen die Blutpumpe 6, die Dialysierflüssigkeitspumpe 16 und die Ultrafiltrationspumpe 23 sowie die Absperrorgane 17 bis 20 ansteuert. Zur Bilanzierung von frischer und verbrauchter Dialysierflüssigkeit steuert die Steuereinheit 25 die Pumpen 16 und 23 und die Absperrorgane 17 bis 20 wie folgt an, um die Dialysevorrichtung in aufeinanderfolgenden Zyklen betreiben zu können, die jeweils zwei Arbeitstakte umfassen.

In dem ersten Arbeitstakt wird die Bilanzkammer 9 unter Verdrängung von verbrauchter Dialysierflüssigkeit, mit der die Bilanzkammer 9 in einem vorausgehenden Arbeitszyklus befüllt worden ist, mit frischer Dialysierflüssigkeit befüllt, während in dem zweiten Arbeitstakt die Bilanzkammer 9 mit verbrauchter Dialysierflüssigkeit unter Verdrängung von frischer Dialysierflüssigkeit befüllt wird. Dabei wird proportionsweise frische und verbrauchte Dialysierflüssigkeit der Dialysierflüssigkeitskammer 4 des Dialysators 1 zugeführt bzw. aus dem Dialysator 1 abgeführt, wodurch frische gegen verbrauchte Dialysierflüssigkeit bilanziert wird.

Die Bilanziervorrichtung verfügt über eine Überwachungseinrichtung A zur Erkennung einer fehlerhaften Bilanzierung. Die Überwachungseinrichtung A weist eine Wiegeeinrichtung 26 und eine Recheneinheit 27 auf, wobei die Recheneinheit 27 über eine Datenleitung 28 mit der Steuereinheit 25 und eine Datenleitung 29 mit der Wiegeeinrichtung 26 verbunden ist. Die Recheneinheit 27 ist über eine Datenleitung 30 mit einer Alarmeinheit 31 verbunden, die einen akustischen und/oder optischen Alarm erzeugt.

Die Wiegeeinrichtung 26 umfasst eine Waage 26A zum Wiegen des Behältnisses 10. Die Recheneinheit 27 empfängt die Messwerte der Waage 26A, um das Gewicht des Behältnisses 10 kontinuierlich zu überwachen. Die Recheneinheit 27 ist derart konfiguriert, dass das Gewicht des Behältnisses 10 überwacht wird. Wenn sich der Betrag des Gewichts des Behältnisses 10 innerhalb eines vorgegebenen Zeitintervalls um einen Betrag ändert, der größer als ein vorgegebener Grenzwert ist, vorzugsweise >0 ist, erzeugt die Recheneinheit 27 ein Steuersignal. Wenn die Recheneinheit 27 ein Steuersignal erzeugt, gibt die Alarmeinheit 31 einen Alarm. Die Steuereinheit 25 empfängt das Steuersignal der Recheneinheit, so dass die Steuereinheit einen Eingriff in die Maschinensteuerung vornehmen kann.

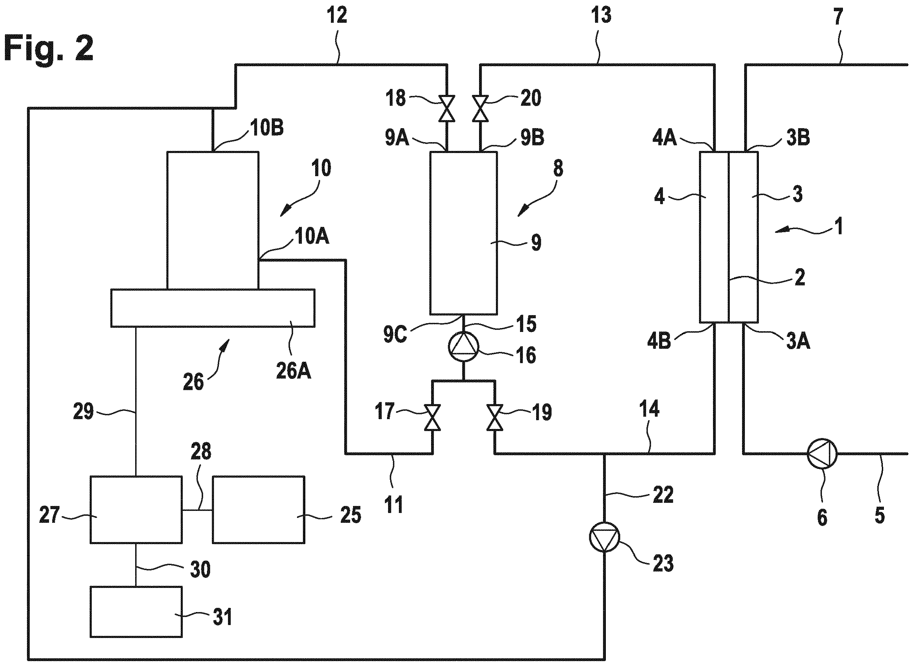

Figur 2 zeigt eine alternative, nicht erfindungsgemäße Ausführungsform der Blutbehandlungsvorrichtung, die sich von der Blutbehandlungsvorrichtung von Fig. 1 nur dadurch unterscheidet, dass die Ultrafiltrationsleitung 22 der Ultrafiltrationseinrichtung 21 nicht zu einem Ablauf 24 führt, sondern an den Einlass 10B des Behältnisses 10 angeschlossen ist, so dass das Ultrafiltrat dem Behältnis 10 zugeführt wird. Die einander entsprechenden Teile sind mit den gleichen Bezugszeichen versehen.
Wenn dem Patienten in einem vorgegebenen Zeitintervall eine vorgegebene Menge an Ultrafiltrat mit der Ultrafiltrationspumpe 23 entzogen wird, nimmt das Gewicht des Behältnisses 10 zu, da das Volumen der Flüssigkeit in dem Behältnis zunimmt. Die Recheneinheit 27 ist derart konfiguriert, dass auf eine fehlerhafte Bilanzierung geschlossen wird, wenn sich das Gewicht des Behältnisses 10 nicht um einen Betrag ändert, der dem Gewicht des Volumens des in dem vorgegebenen Zeitintervall entzogenen Ultrafiltrats entspricht. Wenn dies der Fall ist, wird von der Recheneinheit 27 das Steuersignal erzeugt, wobei Alarm und/oder die Vornahme eines Eingriffs in die Maschinensteuerung ausgelöst wird.

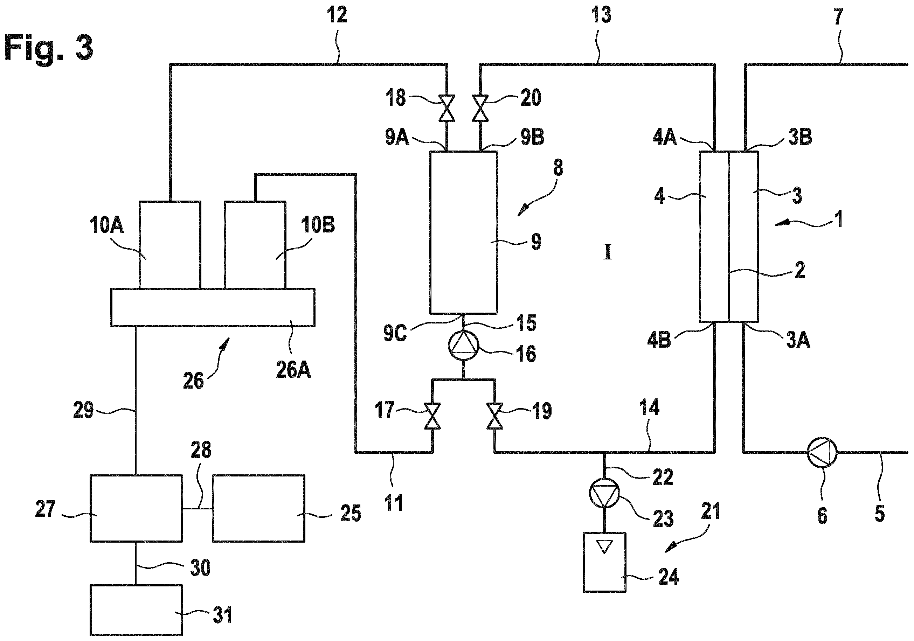

Fig. 3 zeigt ein weiteres, nicht erfindungsgemäßes Ausführungsbeispiel der Dialysevorrichtung, die sich von den Ausführungsformen von Fig. 1 und Fig. 2 nur dadurch unterscheidet, dass anstelle eines einzigen Behältnisses 10 für frische und gebrauchte Dialysierflüssigkeit jeweils ein erstes und eine zweites Behältnis 10A bzw. 10B vorgesehen ist, das mit der ersten Zufluss- bzw. Abflussleitung 11, 12 verbunden ist. Die einander entsprechenden Teile sind wieder mit den gleichen Bezugszeichen versehen.
Die Recheneinheit 27 ist derart konfiguriert, dass das Steuersignal erzeugt wird, wenn in einem vorgegebenen Zeitintervall sich die Summe der Gewichte beider auf der einzigen Waage 26A stehenden Behältnisse 10A, 10B um einen Betrag ändert, der größer als ein vorgegebener Grenzwert ist, vorzugsweise >0 ist. Auch bei diesem Ausführungsbeispiel kann das Ultrafiltrat dem zweiten Behältnis 10B zugeführt werden, wobei die Recheneinheit derart konfiguriert ist, dass das Steuersignal erzeugt wird, wenn das Gewicht des zweiten Behältnisses 10B nicht um einen Betrag von dem Gewicht des ersten Behältnisses abweicht, der dem Gewicht des Volumens des in dem vorgegebenen Zeitintervalls entzogenen Ultrafiltrats entspricht.

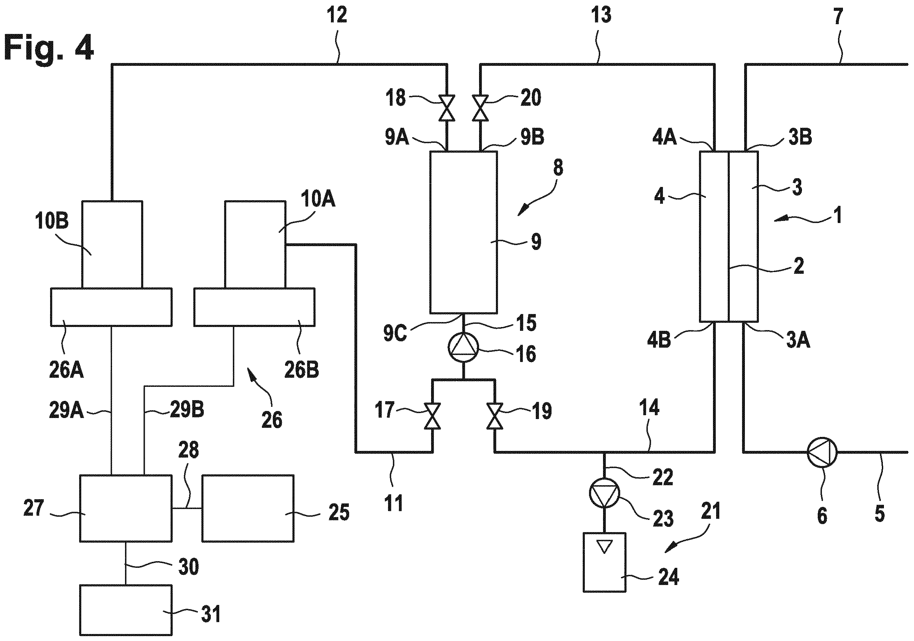

Fig. 4 zeigt eine erfindungsgemäße Dialysevorrichtung mit einer alternativen Überwachungseinrichtung A, die sich von der Überwachungseinrichtung von Fig. 3 nur dadurch unterscheidet, dass die Wiegeeinrichtung 26 für jedes der beiden Behältnisse 10A, 10B eine separate Waage 26A, 26B aufweist. Die einander entsprechen Teile sind wieder mit den gleichen Bezugszeichen versehen. Beide Waagen 26A und 26B sind jeweils über eine Datenleitung 29A, 29B mit der Recheneinheit 27 verbunden. Die Recheneinheit 27 ist derart konfiguriert, dass auf eine fehlerhafte Bilanzierung geschlossen und das Steuersignal erzeugt wird, wenn in einem vorgegebenen Zeitintervall das Gewicht des ersten Behältnisses 10A um einen Betrag von dem Gewicht des zweiten Behältnisses 10B abweicht, der größer als ein vorgegebener Grenzwert ist, vorzugsweise >0 ist. Auch bei dieser Ausführungsform kann das Ultrafiltrat wieder einem der Behältnisse zugeführt werden.

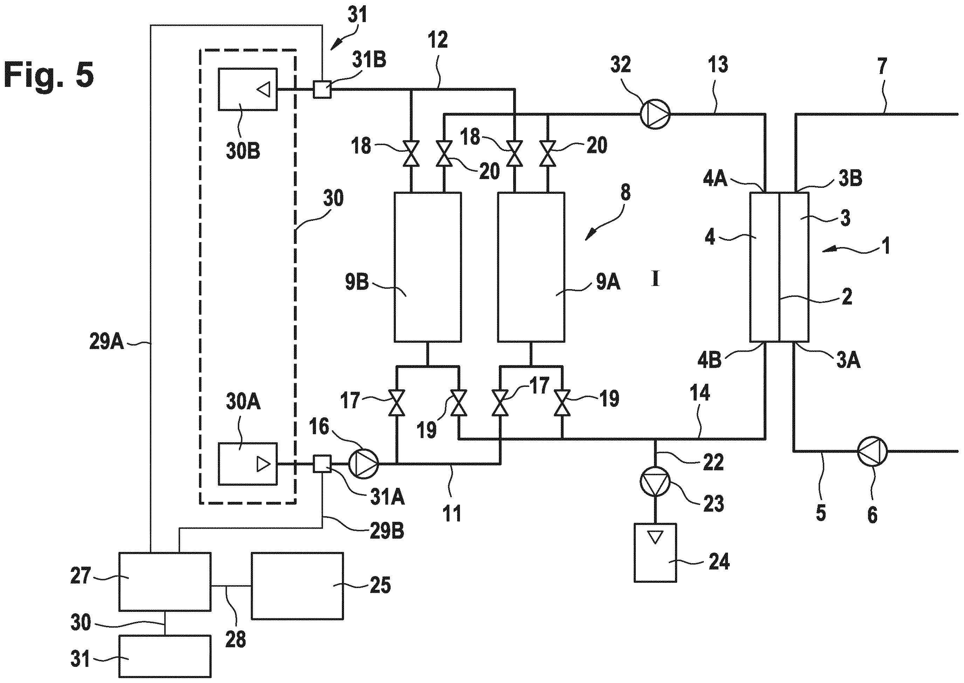

Fig. 5 zeigt eine weitere, erfindungsgemäße Ausführungsform der Dialysevorrichtung. Die einander entsprechenden Teile sind wieder mit den gleichen Bezugszeichen versehen. Die Dialysevorrichtung von Fig. 5 weist eine Bilanziereinheit 8 auf, die über zwei parallel geschaltete Bilanzkammern 9A, 9B verfügt, die wechselweise mit frischer bzw. gebrauchter Dialysierflüssigkeit befüllt werden, um einen kontinuierlichen Dialysierflüssigkeitsfluss durch die Dialysierflüssigkeitskammer 4 des Dialysators 1 aufrechterhalten zu können. Derartige Bilanzkammersysteme gehören zum Stand der Technik. Darüber hinaus weist die Dialysevorrichtung eine zweite Dialysierflüssigkeitspumpe 32 auf, die in der zweiten Abflussleitung 13 angeordnet ist, um Dialysierflüssigkeit in dem inneren Kreislauf I zu fördern.

Die Dialysevorrichtung von Fig. 5 unterscheidet sich von den Dialysevorrichtungen von den Fig. 1 bis 4 weiterhin dadurch, dass frische Dialysierflüssigkeit nicht in einem Behältnis bereitgestellt bzw. gesammelt wird, sondern frische Dialysierflüssigkeit von einer zentralen Versorgungseinrichtung 30 zugeführt bzw. abgeführt wird, die in Fig. 5 nur schematisch dargestellt ist. Die Versorgungseinrichtung 30 weist eine mit der Zuflussleitung 11 verbundene Dialysierflüssigkeitsquelle 30A und einen mit der Abflussleitung 12 verbundenen Abfluss 30B auf. Bei dem Ausführungsbeispiel von Fig. 5 ist anstelle einer Wiegeeinrichtung eine Durchfluss-Messeinrichtung 31 vorgesehen, die einen ersten Durchfluss-Sensor 31A in der ersten Zuflussleitung 11 und einen zweiten Durchfluss-Sensor 31B in der ersten Abflussleitung 12 aufweist. Die Recheneinheit 27 empfängt die mit den Sensoren 31A, 31B gemessenen Flussraten der zu- bzw. abfließenden Dialysierflüssigkeit über die Datenleitungen 29A, 29B. Die Recheneinheit 27 ist derart konfiguriert, dass auf eine fehlerhafte Bilanzierung geschlossen und das Steuersignal erzeugt wird, wenn der Durchfluss in der ersten Zuflussleitung 11 um einen vorgegebenen Betrag von dem Durchfluss in der ersten Abflussleitung 12 abweicht, wobei der vorgegebene Betrag vorzugsweise 0 ist. Wenn die Differenz der beiden Messwerte hingegen 0 ist, kann davon ausgegangen werden, dass die Bilanzierung in dem inneren Flüssigkeitskreislauf exakt ist. Auch bei diesem Ausführungsbeispiel kann das in einem vorgegeben Zeitintervall entzogene Volumen an Ultrafiltrat in die Überwachung des Durchflusses der abgeführten Flüssigkeit mit einbezogen werden.

## Patentansprüche

1. Vorrichtung zum Bilanzieren von Flüssigkeiten für eine extrakorporale Blutbehandlungsvorrichtung mit einer Blutbehandlungseinheit, wobei die Vorrichtung zum Bilanzieren aufweist:
eine Bilanziereinheit (8),
eine zu der Bilanziereinheit (8) führende erste Zuflussleitung (11) zum Zuführen von Flüssigkeit in die Bilanziereinheit und eine von der Bilanziereinheit abgehende erste Abflussleitung (12) zum Abführen von Flüssigkeit aus der Bilanziereinheit,
eine von der Bilanziereinheit (8) abgehende zweite Abflussleitung (13) zum Fördern von Flüssigkeit aus der Bilanziereinheit in die Blutbehandlungseinheit und eine zu der Bilanziereinheit führenden zweite Zuflussleitung (14) zum Fördern von Flüssigkeit aus der Blutbehandlungseinheit in die Bilanziereinheit, wobei
die Vorrichtung zum Bilanzieren eine Überwachungseinrichtung (A) zur Erkennung einer fehlerhaften Bilanzierung von der Flüssigkeit, die der Blutbehandlungseinheit (1) zugeführt wird, und der Flüssigkeit, die aus der Blutbehandlungseinheit abgeführt wird, aufweist,
wobei die Überwachungseinrichtung (A) eine Recheneinheit (27) aufweist, die derart konfiguriert ist,
dass das Volumen oder eine mit dem Volumen korrelierende Größe der Flüssigkeit, die der Bilanziereinheit (8) zugeführt wird, mit dem Volumen oder einer mit dem Volumen korrelierenden Größe der Flüssigkeit, die aus der Bilanziereinheit abgeführt wird, ins Verhältnis gesetzt wird, und
dass auf der Grundlage des Verhältnisses des Volumens oder der mit dem Volumen korrelierenden Größe von der Bilanziereinheit (8) zugeführter und abgeführter Flüssigkeit auf eine fehlerhafte Bilanzierung geschlossen wird, und
wobei die Überwachungseinrichtung (A) eine Wiegeeinrichtung (26) zur Bestimmung der mit dem Volumen korrelierenden Größe der Flüssigkeit, die der Bilanziereinheit (8) zugeführt wird, und der Flüssigkeit, die aus der Bilanziereinheit abgeführt wird, aufweist, und
wobei die Vorrichtung zum Bilanzieren ein mit der ersten Zuflussleitung (11) verbundenes erstes Behältnis (10A) zur Aufnahme der Flüssigkeit, die der Bilanziereinheit (8) zugeführt wird, und ein mit der ersten Abflussleitung (12) verbundenes zweites Behältnis (10B) zur Aufnahme der Flüssigkeit, die aus der Bilanziereinheit abgeführt wird, aufweist,
**dadurch gekennzeichnet, dass**
die Wiegeeinrichtung (26) eine erste Waage (26A) zum Wiegen des ersten Behältnisses (10A) und eine zweite Waage (26B) zum Wiegen des zweiten Behältnisses (10B) aufweist und die Recheneinheit (27) der Überwachungseinrichtung derart konfiguriert ist, dass auf eine fehlerhafte Bilanzierung geschlossen wird, wenn in einem vorgegebenen Zeitintervall das Gewicht des ersten Behältnisses (10A) um einen Betrag von dem Gewicht des zweiten Behältnisses (10B) abweicht, der größer als ein vorgegebener Grenzwert ist.

2. Vorrichtung zum Bilanzieren nach Anspruch 1, **dadurch gekennzeichnet, dass** die Vorrichtung zum Bilanzieren eine Ultrafiltrationseinrichtung (21) aufweist, die eine von der zweiten Abflussleitung (14) abzweigende Ultrafiltrations-Leitung (22) und eine Ultrafiltrations-Pumpe (23) zum Abziehen eines vorgegebenen Volumens an Ultrafiltrat in einem vorgegebenen Zeitintervall von der zweite Abflussleitung aufweist, wobei die Ultrafiltrations-Leitung (22) mit dem zweiten Behältnis (10B) verbunden ist.

3. Vorrichtung zum Bilanzieren von Flüssigkeiten für eine extrakorporale Blutbehandlungsvorrichtung mit einer Blutbehandlungseinheit, wobei die Vorrichtung zum Bilanzieren aufweist:
eine Bilanziereinheit (8),
eine zu der Bilanziereinheit (8) führende erste Zuflussleitung (11) zum Zuführen von Flüssigkeit in die Bilanziereinheit und eine von der Bilanziereinheit abgehende erste Abflussleitung (12) zum Abführen von Flüssigkeit aus der Bilanziereinheit,
eine von der Bilanziereinheit (8) abgehende zweite Abflussleitung (13) zum Fördern von Flüssigkeit aus der Bilanziereinheit in die Blutbehandlungseinheit und eine zu der Bilanziereinheit führenden zweite Zuflussleitung (14) zum Fördern von Flüssigkeit aus der Blutbehandlungseinheit in die Bilanziereinheit, wobei
die Vorrichtung zum Bilanzieren eine Überwachungseinrichtung (A) zur Erkennung einer fehlerhaften Bilanzierung von der Flüssigkeit, die der Blutbehandlungseinheit (1) zugeführt wird, und der Flüssigkeit, die aus der Blutbehandlungseinheit abgeführt wird, aufweist,
wobei die Überwachungseinrichtung eine Recheneinheit (27) aufweist, die derart konfiguriert ist,
dass das Volumen oder eine mit dem Volumen korrelierende Größe der Flüssigkeit, die der Bilanziereinheit (8) zugeführt wird, mit dem Volumen oder einer mit dem Volumen korrelierenden Größe der Flüssigkeit, die aus der Bilanziereinheit abgeführt wird, ins Verhältnis gesetzt wird, und
dass auf der Grundlage des Verhältnisses des Volumens oder der mit dem Volumen korrelierenden Größe von der Bilanziereinheit (8) zugeführter und abgeführter Flüssigkeit auf eine fehlerhafte Bilanzierung geschlossen wird,
**dadurch gekennzeichnet, dass**
die Überwachungseinrichtung (A) eine Durchfluss-Messeinrichtung (30) zur Bestimmung des Volumens der Flüssigkeit, die der Bilanziereinheit (8) zugeführt wird, und der Flüssigkeit, die aus der Bilanziereinheit abgeführt wird, aufweist,
wobei die Durchfluss-Messeinrichtung (31) einen ersten Durchfluss-Sensor (31A) in der ersten Zuflussleitung (11) und einen zweiten Durchfluss-Sensor (31B) in der ersten Abflussleitung (12) aufweist und die Recheneinheit (27) der Überwachungseinrichtung derart konfiguriert ist, dass auf eine fehlerhafte Bilanzierung geschlossen wird, wenn der Durchfluss in der ersten Zuflussleitung (11) um einen vorgegebenen Betrag von dem Durchfluss in der ersten Abflussleitung (12) abweicht.

4. Vorrichtung zum Bilanzieren nach einem der Ansprüche 1 bis 3, **dadurch gekennzeichnet, dass** die Vorrichtung zum Bilanzieren aufweist:
Mittel (16) zum Fördern von Flüssigkeit in die und/oder aus der Bilanziereinheit (8) und Mittel (17-20) zum Unterbrechen des Zuflusses von Flüssigkeit in die Bilanziereinheit (8) und/oder Abflusses von Flüssigkeit aus der Bilanzeinheit und
eine Steuereinheit (25) zum Ansteuern der Mittel (16) zum Fördern von Flüssigkeit und der Mittel (17-20) zum Unterbrechen des Zuflusses und/oder Abflusses von Flüssigkeit.

5. Vorrichtung zur extrakorporalen Blutbehandlung mit einer Blutbehandlungseinheit, **dadurch gekennzeichnet, dass** die Vorrichtung zur extrakorporalen Blutbehandlung eine Vorrichtung zum Bilanzieren von Flüssigkeiten nach einem der Ansprüche 1 bis 4 aufweist.

6. Vorrichtung zur extrakorporalen Blutbehandlung nach Anspruch 5, **dadurch gekennzeichnet, dass** die Vorrichtung zur extrakorporalen Blutbehandlung eine Dialysevorrichtung ist, wobei die Blutbehandlungseinheit ein Dialysator ist.

## Claims

1. Device for balancing fluids for an extracorporeal blood treatment device with a blood treatment device, wherein the device for balancing comprises:
a balancing unit (8),
a first inflow line (11) leading to the balancing unit (8) for delivering fluid to the balancing unit and a first discharge line (12) leading from the balancing unit for discharging fluid from the balancing unit,
a second discharge line (13) leading from the balancing unit (8) for delivering fluid from the balancing unit to the blood treatment device und a second inflow line (14) leading to the balancing unit for delivering fluid from the blood treatment device to the balancing unit,
the device for balancing comprising a monitoring device (A) for detecting an improper balance of the fluid that is delivered to the blood treatment device (1) and the fluid that is discharged from the blood treatment device,
the monitoring device comprising an arithmetic unit (27) that is configured such
that the volume or quantity correlating to the volume of fluid that is delivered to the balancing unit (8) is put in a ratio to the volume or quantity correlating to the volume of fluid that is discharged from the balancing unit, and
that an improper balance is inferred on the basis of the ratio of the volume or quantity correlating to the volume of fluid delivered to and discharged from the balancing unit (8), and
the monitoring device (A) comprising a weighing device (26) for determining the quantity correlating to the volume of fluid that is delivered to the balancing unit (8) and of the fluid that is discharged from the balancing unit, and
the device for balancing comprising a first container (10A) connected to the first inflow line (11) for receiving fluid that is delivered to the balancing unit (8) and a second container (10B) connected to the second discharge line (12) for receiving the fluid that is discharged from the balancing unit,
**characterized in that**
the weighing device (26) has a first scale (26A) for weighing the first container (10A) and a second scale (26B) for weighing the second container (10B), and the arithmetic unit (27) of the monitoring device is configured such that an improper balance is inferred if the weight of the first container (10A) differs from the weight of the second container in a predetermined time interval by an amount that is greater than a predetermined threshold.

2. Device for balancing as set forth in claim 1, **characterized in that** the device for balancing comprises an ultrafiltration device (21) that comprises an ultrafiltration line (22) branching off from the second discharge line (14) and an ultrafiltration pump (23) for withdrawing a predetermined volume of ultra-filtrate in a predetermined time interval from the second discharge line, the ultrafiltration line (22) being connected to the second container (10B).

3. Device for balancing fluids for an extracorporeal blood treatment device with a blood treatment device, wherein the device for balancing comprises:
a balancing unit (8),
a first inflow line (11) leading to the balancing unit (8) for delivering fluid to the balancing unit and a first discharge line (12) leading from the balancing unit for discharging fluid from the balancing unit,
a second discharge line (13) leading from the balancing unit (8) for delivering fluid from the balancing unit to the blood treatment device und a second inflow line (14) leading to the balancing unit for delivering fluid from the blood treatment device to the balancing unit,
the device for balancing comprising a monitoring device (A) for detecting an improper balance of the fluid that is delivered to the blood treatment device (1) and the fluid that is discharged from the blood treatment device,
the monitoring device having an arithmetic unit (27) that is configured such
that the volume or quantity correlating to the volume of fluid that is delivered to the balancing unit (8) is put in a ratio to the volume or quantity correlating to the volume of fluid that is discharged from the balancing unit, and
that an improper balance is inferred on the basis of the ratio of the volume or quantity correlating to the volume of fluid delivered to and discharged from the balancing unit (8),
**characterized in that**
the monitoring device (A) comprises a throughput metering device (30) for determining the volume of fluid that is delivered to the balancing unit (8) and of the fluid that is discharged from the balancing unit, wherein the throughput metering device (31A) comprises a first throughput sensor in the first inflow line (11) and a second throughput sensor (31B) in the first discharge line (12), and the arithmetic unit (27) of the monitoring device is configured such that an improper balance is inferred if the throughput in the first inflow line (11) deviates by a predetermined amount from the throughput in the first discharge line (12).

4. Device for balancing as set forth in any one of claims 1 to 3, **characterized in that** the device for balancing comprises:
means (16) for delivering fluid to and/or from the balancing unit (8) and means (17-20) for interrupting the inflow of fluid into the balancing unit (8) and/or outflow of fluid from the balancing unit, and
a control unit (25) for controlling the means (16) for delivering fluid and the means (17-20) for interrupting the inflow and/or outflow of fluid.

5. Device for extracorporeal blood treatment with a blood treatment device, **characterized in that** device for extracorporeal blood treatment comprises a device for balancing fluids as set forth in any one of claims 1 to 4.

6. Device for extracorporeal blood treatment as set forth in claim 5, **characterized in that** the device for extracorporeal blood treatment is a dialysis apparatus, the blood treatment device being a dialyzer.

## Revendications

1. Dispositif d'équilibrage de liquides pour un dispositif de traitement du sang extracorporel avec une unité de traitement du sang, dans lequel le dispositif d'équilibrage présente :
une unité d'équilibrage (8),
un premier conduit de flux entrant (11) menant à l'unité d'équilibrage (8), servant à amener du liquide dans l'unité d'équilibrage et un premier conduit de flux sortant (12) partant de l'unité d'équilibrage, servant à évacuer du liquide de l'unité d'équilibrage,
un deuxième conduit de flux sortant (13) partant de l'unité d'équilibrage (8), servant à refouler du liquide hors de l'unité d'équilibrage dans l'unité de traitement du sang, et
un deuxième conduit de flux entrant (14) menant à l'unité d'équilibrage, servant à refouler du liquide hors de l'unité de traitement du sang dans l'unité d'équilibrage, dans lequel
le dispositif d'équilibrage présente un système de surveillance (A) servant à identifier un équilibrage erroné du liquide, qui est amené à l'unité de traitement du sang (1), et le liquide, qui est évacué de l'unité de traitement du sang,
dans lequel le système de surveillance (A) présente une unité de calcul (27), qui est configurée de telle manière
que le volume ou une grandeur, en corrélation avec le volume, du liquide, qui est amené à l'unité d'équilibrage (8), est mis en relation avec le volume ou une grandeur, en corrélation avec le volume, du liquide, qui est évacué de l'unité d'équilibrage, et
qu'il est conclu à un équilibrage erroné sur la base du rapport du volume ou de la grandeur, en corrélation avec le volume, du liquide amené à l'unité d'équilibrage (8) et évacué, et
dans lequel le système de surveillance (A) présente un système de pesée (26) servant à définir la grandeur, en corrélation avec le volume, du liquide, qui est amené à l'unité d'équilibrage (8), et le liquide, qui est évacué de l'unité d'équilibrage, et
dans lequel le dispositif d'équilibrage présente un premier récipient (10A) relié au premier conduit de flux entrant (11), servant à recevoir le liquide, qui est amené à l'unité d'équilibrage (8), et un deuxième récipient (10B) relié au premier conduit de flux sortant (12), servant à recevoir le liquide, qui est évacué de l'unité d'équilibrage,
**caractérisé en ce que**
le système de pesée (26) présente une première balance (26A) servant à peser le premier récipient (10A) et une deuxième balance (26B) servant à peser le deuxième récipient (10B), et l'unité de calcul (27) du système de surveillance est configurée de telle manière qu'il est conclu à un équilibrage erroné quand dans un intervalle de temps spécifié, le poids du premier récipient (10A) diverge du poids du deuxième récipient (10B) d'un montant, qui est supérieur à une valeur limite spécifiée.

2. Dispositif d'équilibrage selon la revendication 1, **caractérisé en ce que** le dispositif d'équilibrage présente un système d'ultrafiltration (21), qui présente un conduit d'ultrafiltration (22) bifurquant du deuxième conduit de flux sortant (14) et une pompe d'ultrafiltration (23) servant à retirer un volume spécifié en ultrafiltrat dans un intervalle de temps spécifié du deuxième conduit de flux sortant, dans lequel le conduit d'ultrafiltration (22) est relié au deuxième récipient (10B).

3. Dispositif d'équilibrage de liquides pour un dispositif de traitement du sang extracorporel avec une unité de traitement du sang, dans lequel le dispositif d'équilibrage présente :
une unité d'équilibrage (8),
un premier conduit de flux entrant (11) menant à l'unité d'équilibrage (8), servant à amener du liquide dans l'unité d'équilibrage et un premier conduit de flux sortant (12) partant de l'unité d'équilibrage, servant à évacuer du liquide de l'unité d'équilibrage,
un deuxième conduit de flux sortant (13) partant de l'unité d'équilibrage (8), servant à refouler du liquide hors de l'unité d'équilibrage dans l'unité de traitement du sang, et
un deuxième conduit de flux entrant (14) menant à l'unité d'équilibrage, servant à refouler du liquide hors de l'unité de traitement du sang dans l'unité d'équilibrage, dans lequel
le dispositif d'équilibrage présente un système de surveillance (A) servant à identifier un équilibrage erroné du liquide, qui est amené à l'unité de traitement du sang (1), et le liquide, qui est évacué de l'unité de traitement du sang,
dans lequel le système de surveillance présente une unité de calcul (27), qui est configurée de telle manière
que le volume ou une grandeur, en corrélation avec le volume, du liquide, qui est amené à l'unité d'équilibrage (8), est mis en relation avec le volume ou une grandeur, en corrélation avec le volume, du liquide, qui est évacué de l'unité d'équilibrage, et
qu'il est conclu à un équilibrage erroné sur la base du rapport du volume ou de la grandeur, en corrélation avec le volume, du liquide amené à l'unité d'équilibrage (8) et évacué,
**caractérisé en ce que**
le système de surveillance (A) présente un système de mesure de débit (30) servant à définir le volume du liquide, qui est amené à l'unité d'équilibrage (8), et le liquide, qui est évacué de l'unité d'équilibrage,
dans lequel le système de mesure de débit (31) présente un premier capteur de débit (31A) dans le premier conduit de flux entrant (11) et un deuxième capteur de débit (31B) dans le premier conduit de flux sortant (12) et l'unité de calcul (27) du système de surveillance est configurée de telle manière qu'il est conclu à un équilibrage erroné quand le débit dans le premier conduit de flux entrant (11) diverge du débit dans le premier conduit de flux sortant (12) d'un montant spécifié.

4. Dispositif d'équilibrage selon l'une quelconque des revendications 1 à 3, **caractérisé en ce que** le dispositif d'équilibrage présente :
des moyens (16) servant à refouler du liquide dans et/ou hors de l'unité d'équilibrage (8) et des moyens (17 - 20) servant à interrompre le flux entrant de liquide dans l'unité d'équilibrage (8) et/ou le flux sortant de liquide hors de l'unité d'équilibrage et une unité de commande (25) servant à piloter les moyens (16) servant à refouler du liquide et les moyens (17 - 20) servant à interrompre le flux entrant et/ou le flux sortant de liquide.

5. Dispositif de traitement du sang extracorporel avec une unité de traitement du sang, **caractérisé en ce que** le dispositif de traitement du sang extracorporel présente un dispositif d'équilibrage de liquides selon l'une quelconque des revendications 1 à 4.

6. Dispositif de traitement du sang extracorporel selon la revendication 5, **caractérisé en ce que** le dispositif de traitement du sang extracorporel est un dispositif de dialyse, dans lequel l'unité de traitement du sang est un dialyseur.
